# EUROPEAN PATENT APPLICATION

(11) **EP 3 884 945 A1**
(43) Date of publication of application: **29.09.2021**
(21) Application number: 19886166.8
(22) Date of filing: 22.11.2019
(51) Int. Cl.: A61K 31/454, A61K 31/56, A61P 37/00, A61P 43/00, A61K 31/375, A61P 11/06, A61P 37/08

(54) **GLUTARIMIDE DERIVATIVE FOR OVERCOMING RESISTANCE TO STERIODS**

(30) Priority: 23.11.2018 RU 2018141291
(71) Applicant: "ChemImmune Therapeutics" Limited Liability Company, Moscow 121205 (RU)
(72) Inventor: NEBOLSIN, Vladimir Evgenievich, Moskovskaya obl., 143581 (RU)
(74) Representative: Goddar, Heinz J.
(86) International application number: PCT/RU2019/050225
(87) International publication number: WO 2020/106191

(57) **Abstract**

The present invention relates to the medicine, in particular, to a new drug effective for the treatment of diseases associated with aberrant interferon gamma signaling, such as Sjogren's syndrome, dermatomyositis, systemic lupus erythematosus, or systemic sclerosis; for the treatment of patients suffering from cough; and for the treatment of disorders in steroid-resistant patients, such as asthma, rheumatoid arthritis, systemic lupus erythematosus, and gastrointestinal diseases, by administering a compound 1-(2-(1H-imidazol-4-yl)ethyl) piperidin-2,6-dione

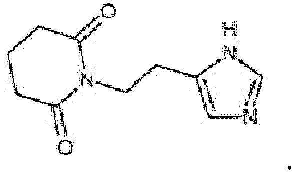

The invention provides the creation of a new effective drug for overcoming steroid resistance.

## Description

### TECHNICAL FIELD OF THE INVENTION

The invention relates to medicine, in particular, to a new drug effective in the treatment of diseases associated with aberrant interferon gamma signaling, such as Sjögren's syndrome, dermatomyositis, systemic lupus erythematosus, or systemic sclerosis, as well as in the treatment of patients suffering from cough, and in the treatment of disorders in steroid-resistant patients, such as asthma, rheumatoid arthritis, systemic lupus erythematosus, and gastrointestinal diseases.

### BACKGROUND

The problem of treating chronic inflammatory diseases is one of the most actual and socially significant problems of modern medicine. The main drugs in pathogenetic therapy of chronic inflammatory diseases are currently corticosteroids - prednisolone, dexamethasone, hydrocortisone, betamethasone and others [Am J Respir Crit Care Med., 2017 Aug 15; 196(4):414-424]. Glucocorticosteroids (GCS) are the most effective drugs for treating bronchial asthma, chronic glomerulonephritis, interstitial nephritis, rheumatoid arthritis; they are used to a lesser extent in the treatment of chronic obstructive bronchitis, autoimmune pancreatitis, and ulcerative necrotizing colitis. Their therapeutic effect is due to a powerful anti-inflammatory effect, which is associated with inhibitory action on inflammatory cells and mediators produced by these cells and includes inhibition of the production of cytokines (interleukins) and pro-inflammatory mediators, and their interaction with target cells [Mediators Inflamm. 1998; 7(4):229-37].

It is important to note that sensitivity to corticosteroid drugs decreases in a significant portion of patients during long-term therapy, i.e. resistance to steroids develops. Low sensitivity to steroid therapy is manifested in the absence of a pronounced therapeutic effect and requires an increase in corticosteroid dose. However, in steroid-resistant patients, an increase in steroid dose provides only a short-time increase in the anti-inflammatory and therapeutic effect. In addition to the emergence of resistance during long-term corticosteroid therapy, the disease is also encountered in clinical practice in steroid-resistant forms, which greatly complicates the selection of drugs for pathogenetic therapy and is the main problem in the treatment of these patients [Curr Allergy Asthma Rep. 2002 Mar; 2(2):144-50] .

Steroid resistance occurs in a variety of inflammatory and autoimmune diseases, including rheumatoid arthritis, systemic lupus erythematosus, and bowel disease [Arthritis Res Ther. 2016 Jun 14; 18(1) :139, and Clin Rheumatol. 2016 May; 35(5) :1367-75]. Steroid resistance is usually local in nature, i.e. it is observed in the area of chronic inflammation. The main possible mechanisms for the development of steroid resistance include a defect in translocation of hormone-receptor complexes from the cytoplasm into the nucleus, an excessive production of "inflammatory" cytokines (in particular, IL-2, IL-4, IL-13), an increased expression of abnormal β-receptors in cells, the binding of the "hormone-receptor" complex to transcription factors (e.g., AP-1), p38 mitogen-activated protein kinase (p38 MAPK)-induced phosphorylation of steroid receptors, and a decrease in histone deacetylase activity [J Steroid Biochem Mol Biol. 2010 May 31; 120 (2-3) :76-85] .

A known method for increasing the therapeutic efficacy of the therapy employed is the administration of steroids in combination with cytostatic drugs to patients with autoimmune diseases [Kotter I., Duck H., Saal J. et al. Therapy of Behcet's disease // Ger. J. Ophthalmol. - 1996. - Vol.5, No. 2. - p.92-97.]. However, such a complex therapy worsens existing side effects (nephrotoxic, hepatotoxic and hematotoxic), which is due to the fact that both groups of drugs (both corticosteroids and cytostatics) have pronounced side effects. The common side effects increase manifold when administered in combination. Sometimes there can be a potentiation of side effects up to the development of toxic crises. Thus, in clinical practice, there is a pressing need for drugs that can overcome steroid resistance.

Asthma and other chronic obstructive pulmonary diseases are among diseases leading in the number of days of incapacity for work, causes of disability in the morbidity structure and are ranked fourth among the causes of death [Clin Chest Med. 2014 Mar; 35(1):7-16., Eur Respir J. 2001 May; 17(5):982-94.]. Daily administration of inhaled glucocorticosteroids remains the 'gold standard' therapy for asthma and is effective for most patients. However, some patients with severe disease need the use of oral glucocorticosteroids. Nevertheless, some patients remain unresponsive to therapy, despite of high doses of oral glucocorticosteroids used [Lancet. 2010. V. 376. P.814-825]. These steroid-insensitive patients generally have no signs of eosinophilic inflammation [Froidure Eur Respir J 2016; 47:304-319]. Patients with a non-eosinophilic asthma phenotype respond significantly worse to therapy with inhaled corticosteroids than patients with an eosinophilic asthma phenotype; this difference was confirmed in a clinical study [Thorax. 2007 Dec; 62(12):1043-1049] and allowed the authors to identify non-eosinophilic asthma as a separate steroid-resistance phenotype of the disease [Am J Respir Crit Care Med. 2009. V. 180. P. 388-395]. It is important to note that the costs of steroid-resistant asthma therapy account for approximately 50% of total health care costs for asthma therapy [Curr Drug Targets. 2010 Aug; 11(8):957-70].

The accumulated clinical data have demonstrated that the production of interferon gamma (IFN-γ) and interleukin 17A (IL-17A) by blood cells in asthmatic patients may be a predictor of steroid resistance. In [J Allergy Clin Immunol. 2015 Sep; 136(3):628-637.e4] the authors investigated the predictive potential of IFN-γ and IL-17A levels to determine steroid resistance. The study showed that the levels of IFN-γ, IL-17A, and IFN-+IL-17A negatively correlated with the response intensity to glucocorticoid therapy (prednisolone 40 mg for 2 weeks). In addition, peripheral blood mononuclear cells of steroid-resistant asthma patients produce significantly more IFN-γ and IL-17A than blood cells of steroid-sensitive asthma patients [J Allergy Clin Immunol. 2015 Sep; 136(3):628-637.e4]. Additional confirmation of the role of IFN-γ in the development of steroid-resistant asthma was obtained in a murine model of asthma induced by the introduction of OVA-specific cells producing either Th1 cytokines (IL-2, IL-12, IFN-γ) or Th2 cytokines (IL-4, IL-5, IL-13). In the first case, the animals showed the development of corticosteroid-resistant hyperreactivity of the lungs. However, the Th2 model exhibited eosinophilic inflammation that responded well to corticosteroid therapy [J Immunol. 2009 Apr 15; 182(8):5107-15].

Solving the problem of steroid resistance is one of the key challenges in the treatment of inflammatory bowel disease [Am J Physiol Gastrointest Liver Physiol. 2013 Dec; 305(11):G763-85]. The development of chronic inflammation in gastrointestinal tract tissues leads to the influx of interferon gamma-producing cells (mainly macrophages, T cells and NK cells) [Cytokine. 2010 Apr; 50(1):1-14; J Immunol. 1996 Aug 1; 157(3):1261-70]. Clinical data confirm a negative correlation of IFN-γ levels with the response intensity to glucocorticoid therapy. It is important to note that the use of antibodies to IFN-γ does not completely restore the response to therapy in steroid-resistant patients [Gut. 2006 Aug; 55(8):1131-7; Inflamm Bowel Dis. 2010 Feb; 16(2):233-42], which indicates a complex and multifactorial pathogenesis of steroid resistance in inflammatory bowel disease. In particular, it was shown that Th1 and Th17 cells producing excessive amounts of the cytokine IL-17 were involved in the development of steroid resistance and aberrant interferon gamma signaling [Am J Physiol Gastrointest Liver Physiol. 2013 Dec; 305(11):G763-85].

Thus, the literature data allow the conclusion that in clinical practice there is a pronounced need for drugs that can overcome steroid resistance. The overcoming steroid resistance is of great importance for the treatment of chronic obstructive pulmonary disease, and especially for the treatment of non-eosinophilic (steroid-resistant) asthma as well as inflammatory bowel diseases.

Another group of conditions requiring the development of new therapy are diseases associated with aberrant IFN-γ signaling. This group of diseases includes in particular cough hypersensitivity syndrome that is usually developed against the background of upper respiratory tract infections [Allergy Asthma Immunol Res. 2017 Sep; 9(5):394-402; Rev Alerg Mex. 2019 Apr-Jun; 66(2):217-231]. The immune response to a respiratory tract infection results in the influx of T-lymphocytes and aberrant IFN-γ production. Since an excessive production of IFN-γ is associated with the development of chronic cough and cough hypersensitivity syndrome [J Clin Pharm Ther. 2011 Jun; 36(3):416-8], the suppression of aberrant IFN-γ signaling will effectively inhibit cough hypersensitivity syndrome developing against the background of upper respiratory tract infections.

An impaired IFN-γ signaling is characteristic of a number of autoimmune diseases such as Sjögren's syndrome (systemic autoimmune damage to connective tissue) [Proc Natl Acad Sci USA. 2012 Oct 23; 109(43):17609-14], systemic lupus erythematosus, dermatomyositis and systemic sclerosis [Discov Med. 2013 Sep; 16(87):123-131]. The present invention is aimed at solving the above problems.

### SUMMARY OF INVENTION

The objective of the present invention is to develop a new drug effective for the treatment of diseases associated with aberrant interferon gamma signaling, such as Sjögren's syndrome, dermatomyositis, systemic lupus erythematosus, or systemic sclerosis, for the treatment of patients suffering from cough, and for the treatment of disorders in steroid resistant patients, such as asthma, rheumatoid arthritis, systemic lupus erythematosus, and gastrointestinal diseases.

The technical result of the present invention is in increasing the effectiveness of baseline therapy with corticosteroids in steroid-resistant patients.

The specified technical result is achieved by using a compound 1-(2-(1H-imidazol-4-yl)ethyl)piperidine-2,6-dione: 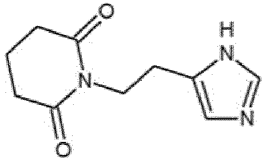 or a pharmaceutically acceptable salt thereof.

The compound 1-(2-(1H-imidazol-4-yl)ethyl)piperidine-2,6-dione is known and described in WO 2014/168522.

One embodiment of the present invention provides use of the compound 1-(2-(1H-imidazol-4-yl)ethyl)piperidine-2,6-dione of the formula: 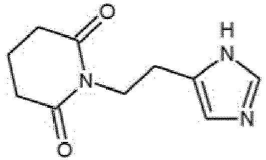 or a pharmaceutically acceptable salt thereof for suppressing aberrant interferon gamma signaling.

Another embodiment of the present invention provides use of Compound 1 for the treatment of a disease associated with aberrant interferon gamma signaling. The disease associated with aberrant interferon gamma signaling is Sjögren's syndrome, dermatomyositis, systemic lupus erythematosus, or systemic sclerosis.

Another embodiment of the present invention provides use of Compound 1 for delying or eliminating the onset of resistance to steroid therapy.

A further embodiment of the present invention provides use of the compound 1-(2-(1H-imidazol-4-yl)ethyl)piperidine-2,6-dione of the formula: 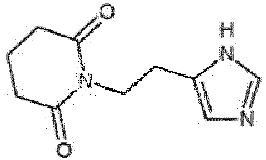 or a pharmaceutically acceptable salt thereof for the treatment of a disorder in steroid-resistant patients. The disorder is asthma, rheumatoid arthritis, systemic lupus erythematosus, gastrointestinal tract diseases, or cough.

Another embodiment of the present invention provides a pharmaceutical composition for suppressing aberrant interferon gamma signaling, comprising a therapeutically effective amount of the compound 1-(2-(1H-imidazol-4-yl)ethyl)piperidine-2,6-dione of the formula: 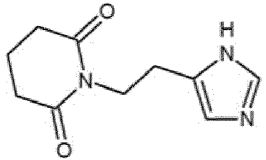 or a pharmaceutically acceptable salt thereof and at least one pharmaceutically acceptable carrier.

Another embodiment of the present invention provides a pharmaceutical composition for treating a disease associated with aberrant interferon gamma signaling. The disease associated with aberrant interferon gamma signaling is asthma, rheumatoid arthritis, systemic lupus erythematosus, gastrointestinal tract diseases, or cough.

Another embodiment of the present invention provides a pharmaceutical composition for preventing resistance to steroid therapy.

Another embodiment of the present invention provides a pharmaceutical composition for treating a disorder in a steroid-resistant patient, comprising a therapeutically effective amount of the compound 1-(2-(1H-imidazol-4-yl)ethyl)piperidine-2,6-dione of the formula: 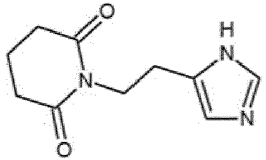 or a pharmaceutically acceptable salt thereof and at least one pharmaceutically acceptable carrier. The disorder associated with aberrant interferon gamma signaling and with the development of steroid resistance is asthma, rheumatoid arthritis, systemic lupus erythematosus, gastrointestinal tract diseases, or cough.

Another embodiment of the present invention provides use of Compound 1 for the preparation of a pharmaceutical composition for suppressing aberrant interferon gamma signaling.

Another embodiment of the present invention provides use of Compound 1 for the preparation of a pharmaceutical composition for the treatment of a disorder in a steroid-resistant patient.

Another embodiment of the present invention provides a method for treating a disease associated with aberrant interferon gamma signaling, comprising administering a therapeutically effective amount of the compound 1-(2-(1H-imidazol-4-yl)ethyl)piperidine-2,6-dione of the formula: 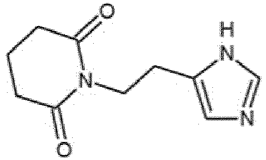 or a pharmaceutically acceptable salt thereof or a pharmaceutical composition comprising thereof to an organism. Another embodiment of the present invention provides a method, wherein Compound 1 is administered at a dose of 10-200 mg/day, preferably 100 mg. Yet another embodiment of the present invention provides a method, wherein Compound 1 is administered 1-2 times a day.

Another embodiment of the present invention provides a method for treating a disorder in a steroid-resistant patient, comprising administering a therapeutically effective amount of a compound 1-(2-(1H-imidazol-4-yl)ethyl)piperidine-2,6-dione of the formula: 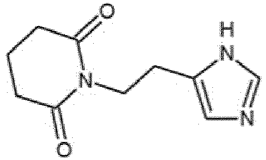 or a pharmaceutically acceptable salt thereof or a pharmaceutical composition comprising thereof to the body. Another embodiment of the present invention provides a method, wherein Compound 1 is administered at a dose of 10-200 mg/day, preferably 100 mg. Yet another embodiment of the present invention provides a method, wherein Compound 1 is administered 1-2 times a day.

Another embodiment of the present invention provides a combination for the treatment of a disorder in a steroid-resistant patient, comprising a therapeutically effective amount of a compound 1-(2-(1H-imidazol-4-yl)ethyl)piperidine-2,6-dione of the formula: 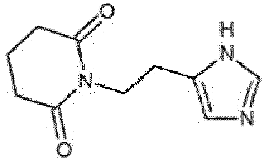 or a pharmaceutically acceptable salt thereof and a therapeutically effective amount of a steroid. The steroid is a corticosteroid, and the disorder is asthma, rheumatoid arthritis, systemic lupus erythematosus, gastrointestinal diseases, or cough.

Another embodiment of the present invention provides a method for treating a disorder in a steroid-resistant patient, comprising administering a combination of Compound 1 and a steroid to the body.

Another embodiment of the present invention is a method, wherein Compound 1 and a steroid are administered simultaneously or separately. Moreover, the specified compound is administered at a dose of 10-200 mg/day, preferably 100 mg. In addition, the compound is administered 1-2 times a day.

### Description of drawings

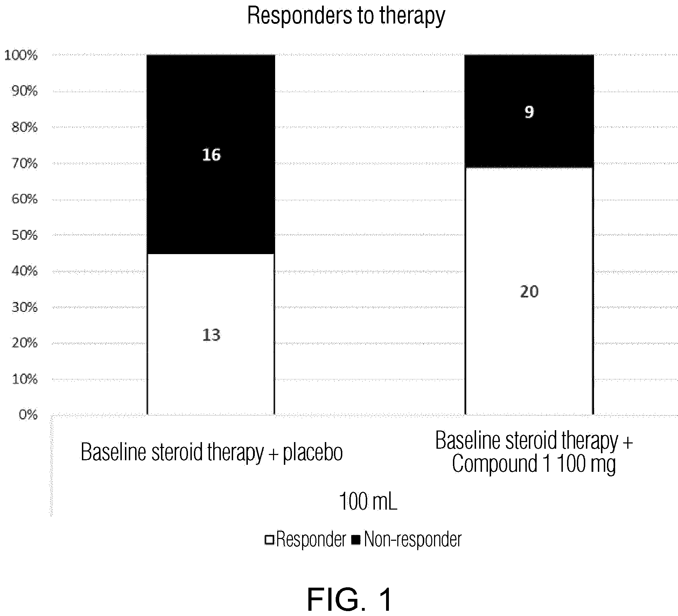

Figure 1. The number of patients who responded to baseline therapy with inhaled glucocorticosteroids against the background of the administration of Compound 1 or placebo.

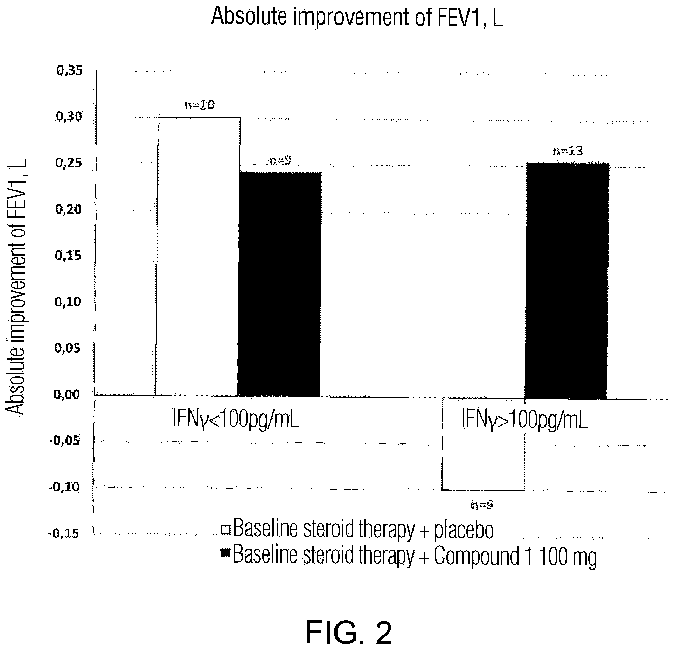

Figure 2. The effect of the IFN-γ level (pg/ml) at the time of trial inclusion on an absolute increase (L) in forced expiratory volume in the first second (FEV1) (week 12 vs. week 0) in patients who received baseline steroid therapy and Compound 1 at a dose of 100 mg (designated as "Baseline steroid therapy + Compound 1, 100 mg") and in patients who received baseline steroid therapy and placebo (designated as "Baseline steroid therapy + placebo"). Compound 1: For each point, n is the number of patients falling into a given subgroup depending on the type of therapy and the IFN-γ level.

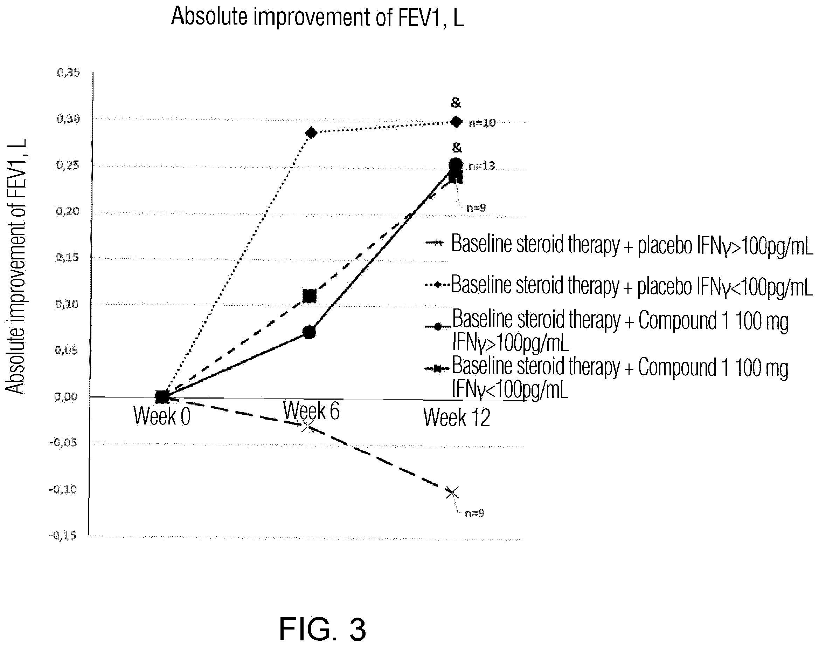

Figure 3. Response to therapy (a change in FEV1 (L), week 12 vs. week 0) in patients who received baseline steroid therapy and Compound 1 at a dose of 100 mg (designated as "Baseline steroid therapy + Compound 1, 100 mg") and in patients who received baseline steroid therapy and placebo (designated as "Baseline steroid therapy + placebo"), depending on the IFN-γ level at the time of inclusion.

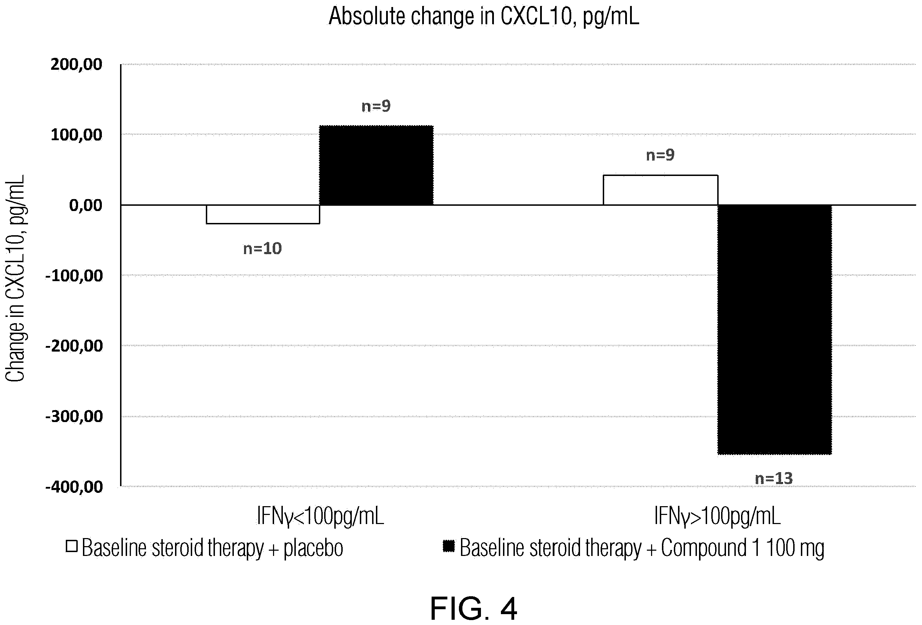

Figure 4. A change in the concentration of interferon-γ-dependent cytokine CXCL10 (interferon-gamma-induced protein IP10) in the patients' blood plasma during administration of Compound 1 or placebo in combination with baseline steroid therapy, depending on the IFN-γ level at the time of inclusion (difference between levels at week 12 and week 0).

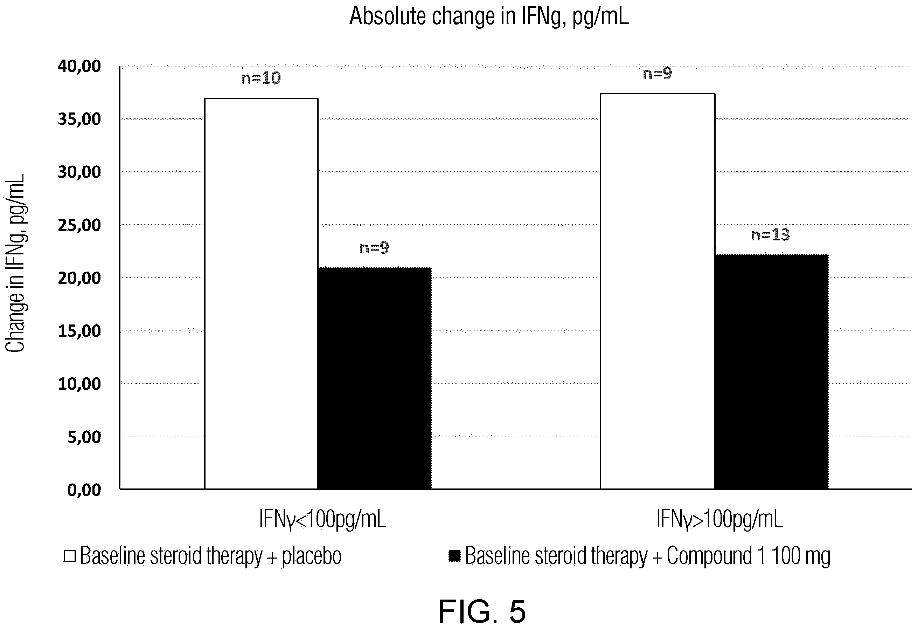

Figure 5. A change in the concentration of interferon-γ in the patients' blood plasma during administration of Compound 1 or placebo in combination with baseline steroid therapy, depending on the IFN-γ level at the time of inclusion (difference between levels at week 12 and week 0).

### DETAILED DISCLOSURE OF THE INVENTION

The preparation of Compound 1, which is the subject matter of the present invention, and a number of other chemical compounds is described in WO 2014/168522. The present patent application describes glutarimide derivatives with antiviral action, their use for the treatment of rhinosinusitis and other upper respiratory tract diseases.

WO 2015/072893 describes the use of Compound 1 for the treatment of diseases associated with the development of eosinophilic inflammation, including eosinophilic asthma. However, the development of eosinophilic inflammation is characteristic mainly of steroid-sensitive forms of asthma, whereas the bronchoalveolar lavage (BAL) in therapeutically resistant patients who received therapy with high doses of systemic corticosteroids showed a large number of neutrophils, i.e. steroid-resistant patients had predominantly neutrophilic inflammation [Turato G., Baraldo S., Zuin R. The laws of attraction: chemokines, neutrophils and eosinophils in severe exacerbations of asthma. Thorax. 2007; 62 (6) :465-466] .

In clinical studies of the activity of Compound 1, which is the subject matter of the present invention, it has been unexpectedly found that the therapeutic use of Compound 1 effectively increases the number of responders to standard corticosteroid therapy and also suppresses aberrant interferon gamma signaling. The overcoming of corticosteroid resistance cannot be predicted or explained by the ability of Compound 1 to exert an antiviral effect or suppress eosinophilic inflammation.

Thus, Compound 1 has a previously unknown pharmacological activity associated with the effect on aberrant interferon gamma signaling and increases the response of patients to corticosteroid therapy, which indicates the potential applicability of Compound 1 for the treatment of diseases associated with aberrant interferon gamma signaling, such as Sjögren's syndrome, dermatomyositis, systemic lupus erythematosus, or systemic sclerosis, for the treatment of patients with cough, and for the treatment of disorders in steroid-resistant patients, such as asthma, rheumatoid arthritis, systemic lupus erythematosus, and gastrointestinal diseases.

### Terms and definitions

The term "glucocorticosteroids" or "glucocorticoids" means steroid hormones from the subclass of corticosteroids and/or their synthetic analogs.

The term "corticosteroids" includes the subclass of steroid hormones and/or synthetic analogs thereof.

The term "Compound 1" refers to a compound 1-(2-(1H-imidazol-4-yl)ethyl)piperidine-2,6-dione, which is also represented by the structural formula:

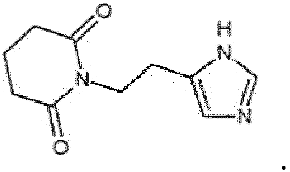

The term "steroid resistance" means a disease condition in which the steroid therapy, which is as a rule effective in the treatment of patients with said disease, is ineffective. Steroid-resistant patients include, but are not limited to, patients who do not or poorly, or insufficiently respond to therapy with systemic or oral corticosteroids according to response routine parameters.

The term "pharmaceutically acceptable salts" or "salts" includes salts of active compounds, prepared with relatively non-toxic acids. Examples of pharmaceutically acceptable non-toxic salts include salts formed with inorganic acids such as hydrochloric, hydrobromic, phosphoric, sulfuric and perchloric acids, or organic acids such as acetic, oxalic, maleic, tartaric, succinic, citric or malonic acid, or prepared by other methods used in this field. Other pharmaceutically acceptable salts include adipate, alginate, ascorbate, aspartate, benzenesulfonate, benzoate, bisulfate, borate, butyrate, camphor, camphorsulfonate, citrate, cyclopentane propionate, digluconate, dodecyl sulfate, ethanesulfonate, formiate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate (mesylate), 2-naphthalenesulfonate, nicotinate, nitrate, oleate, oxalate, palmitate, pamoate, pectinate, persulfate, 3-phenylpropionate, phosphate, picrate, pivalate, propionate, hemi-fumarate, stearate, succinate, sulfate, tartrate, thiocyanate, p-toluenesulfonate (tosylate), undecanate, valerate and the like.

The terms "treatment" and "therapy" encompass the treatment of pathological conditions in mammals, preferably in humans, and include: a) reducing, b) blocking (arresting) the course of a disease, c) alleviating disease severity, i.e. inducing disease regression, d) reversing a disease or condition to which the term applies, or one or more symptoms of the disease or condition.

The terms "prophylaxis" and "prevention" encompass the elimination of risk factors, and prophylactic treatment of subclinical stages of a disease in mammals, preferably in humans, aimed at reducing the likelihood of the occurrence of clinical stages of the disease. The selection of patients for prophylactic therapy is based on factors that are known to be associated with an increased risk of progressing to clinical stages of the disease, compared with general population. Preventive therapy includes a) primary prevention and b) secondary prevention. Primary prevention is defined as prophylactic treatment of patients with a disease that has not reached clinical stage. Secondary prevention is the prevention of recurrence of the same or similar clinical condition of the disease.

### Method for the therapeutic use of compounds

The subject matter of the invention also includes the administration a therapeutically effective amount of a compound according to the invention to a subject in need of appropriate treatment. A therapeutically effective amount means the amount of a compound that when administered or delivered to a patient most likely provides a desired response of the patient to the treatment (prophylaxis). The required exact amount may vary from subject to subject, depending on the age, body weight, and general condition of the patient, disease severity, a method of drug administration, use in combination with other drugs, and the like.

A compound according to the invention or a pharmaceutical composition comprising the compound can be administered to a patient in any amount (preferably, the daily dose of the active substance is up to 0.2 g per patient per day, most preferably the daily dose is 10-200 mg/day, preferably 100 mg) and by any route of administration (preferably by oral route of administration) that is effective for the treatment or prophylaxis of a disease.

After mixing a drug with a specific suitable pharmaceutically acceptable carrier at a desired dosage, compositions according to the invention can be administered to humans or other animals orally, parenterally, topically, and the like.

The administration can be made both once and several times a day, week (or any other time interval), or from time to time. In addition, the compound can be administered to a patient every day for a specified period of days (e.g., 2-10 days), followed by a period without administration (e.g., 1-30 days).

When the compound according to the invention is used as part of a combination therapy, the dose of each of the combination therapy components is administered over a desired treatment period. The compounds of the combination therapy can be administered to the patient's body simultaneously both in the form of a dosage containing all the components and in the form of individual dosages of the components.

### Use of Compound 1 in combination therapy

Although Compound 1 according to the invention can be administered as an individual active pharmaceutical agent, it can also be used in combination with one or more other agents; in particular, the other agent can be a glucocorticosteroid, a leukotriene receptor antagonist, a bronchodilator, a monoclonal antibody, etc. The therapeutic agents, when administered in combination, can be administered in different dosage forms simultaneously or sequentially at different times, or the therapeutic agents can be combined in a single dosage form.

The phrase "combination therapy" as related to the compound of the invention used in combination with other pharmaceutical agents means simultaneous or sequential administration of all agents such that a beneficial effect of the drug combination will be provided in any way. Co-administration implies, in particular, co-delivery, for example, in one tablet, capsule, injection or another form having a fixed ratio of active substances, as well as simultaneous delivery in several, separate dosage forms for each compound, respectively.

Thus, Compound 1 of the invention can be administered in combination with additional therapies known to those skilled in the prevention and treatment of corresponding diseases, including the use of antibacterial, cytostatic and cytotoxic drugs, medical preparations for suppressing symptoms or side effects of one of the drugs.

If the dosage form is a fixed dose, such a combination comprises a compound according to the invention within an acceptable dosage range. Compound 1 of the invention can also be administered to a patient sequentially with other agents, if a combination of these drugs is not possible. The invention is not limited to a certain sequence of administration; the compound of the invention can be administered to a patient concurrently or at any time before or after the administration of another drug.

### Examples

### Preparation of the compound according to the invention

The preparation of Compound 1, which is the subject matter of the present invention, and a number of other chemical compounds, is described in WO 2014/168522. This patent application describes glutarimide derivatives with antiviral action, their use for the treatment of rhinosinusitis and other upper respiratory tract diseases.

### Characterization of the biological activity of the compound according to the invention

The biological activity of Compound 1, which is the subject matter of the present invention, has been studied in extensive preclinical trials, and in a multicenter, double-blind, randomized Phase II clinical trial over a 12-week period of treatment of patients with bronchial asthma. The therapeutic use of Compound 1 has been shown to effectively increase the number of responders to standard therapy with inhaled corticosteroids. The overcoming of resistance to inhaled corticosteroids cannot be predicted or explained by the ability of Compound 1 to exert antiviral effects or suppress eosinophilic inflammation.

### Example 1. Study of the activity of Compound 1 in a clinical trial

In a multicenter, double-blind, randomized, parallel-group Phase II clinical trial study on evaluation of the effectiveness and safety of various doses of Compound 1 over placebo in a 12-week treatment of patients with bronchial asthma (PULM-XC8-02, NCT03450434), it has been unexpectedly found that the therapeutic use of Compound 1 effectively increases the number of responders to standard therapy with inhaled corticosteroids. Thus, Compound 1 is potentially useful for the therapy of diseases associated with the development of steroid resistance, in particular for the treatment of steroid-resistant asthma.

In the clinical trial, eligible patients were randomized 1:1:1:1 to one of four groups:
- Compound 1 at a dose of 2 mg per day;
- Compound 1 at a dose of 10 mg per day;
- Compound 1 at a dose of 100 mg per day; and
- Placebo.

During the study therapy phase, patients received Compound 1 or placebo for 12 weeks against the background of baseline steroid therapy with low doses of inhaled corticosteroids. Compound 1 or placebo was administered orally once a day, 30 minutes before breakfast.

A clinically significant effect was obtained at a dose of Compound 1 of 100 mg per day.

An exploratory analysis of the results of the clinical trial in patients with bronchial asthma showed that Compound 1 effectively increased the number of responders to standard therapy. For example, the use of baseline steroid therapy and placebo led to an increase in FEV1 by 100 ml or more in only 13 patients out of 29, while the use of baseline steroid therapy and Compound 1 resulted in a response in 20 patients out of 29, thus allowing a significant increase in the number patients with a response to baseline steroid therapy (Fig. 1). Thus, one of the effects of the use of Compound 1 is the overcoming of the resistance to corticosteroids, which is the backbone of the baseline therapy of patients enrolled in the clinical trial.

To study the effect of the IFN-γ baseline level in patients with asthma, the results obtained in the clinical study were additionally analyzed; the patients' response to therapy was studied depending on their IFN-γ baseline level in the blood at the time of trial inclusion (determined by the Bio-Plex Pro Human Chemokine Panel Assay (Bio-Rad)).

The patients' response to baseline therapy in the groups of baseline steroid therapy and placebo decreased with an increase in the IFN-γ baseline level (Fig. 2). The use of baseline steroid therapy in patients with an IFN-γ level >100 pg/ml did not lead to a positive change in respiratory function, as determined by a change in FEV1 (an observed decrease of 0.1 L or more, Fig. 2), i.e. these patients showed resistance to steroid therapy (the lack of positive response to therapy). The use of Compound 1 at a dose of 100 mg per day administered against the background of baseline steroid therapy provided a significantly greater and clinically significant response to therapy in patients, especially in those with an IFN-γ level more than 100 pg/ml (Fig. 3). This fact indicates the overcoming of steroid resistance that can be caused, inter alia, by aberrant IFN-γ signaling.

Moreover, in the clinical trial, Compound 1 was also analyzed for its effect on IFN-γ signal transduction. It was shown that Compound 1 administered against the background of baseline steroid therapy suppressed the concentration of interferon-γ-dependent cytokine CXCL10 (interferon-gamma-induced protein IP10) in patients with an IFN-γ baseline level >100 pg/ml, while the group of patients received placebo against the background of baseline steroid therapy had a slight increase in the level of CXCL10 (Figure 4). In addition, the therapeutic use of Compound 1 against the background of baseline therapy also led to negative dynamics of IFN-γ concentrations in the blood plasma of patients compared with the use of only baseline therapy and placebo, regardless of the IFN-γ level at the time of inclusion (Figure 5). Thus, the suppression of steroid resistance induced by the use of Compound 1 could potentially be associated with an effect on the aberrant activity (signaling) of IFN-γ.

### Example 2. Study of the activity of Compound 1 in a model of acute oxazolone-induced intestinal inflammation

The activity of Compound 1 in a model of acute oxazolone-induced ulcerative colitis was studied using the standard method [Immunity. 2002. P. 629-638].

In the study, female balb/c mice (6-8 weeks old) were used. Compound I was administered intragastrically, three times: 1 hour, 25 hours, and 49 hours after rectal administration of oxazolone. The body weight of the animals was measured before and 24, 48 and 72 hours after administration of oxazolone. The intestinal wall damage was evaluated under a microscope 72 hours after administration of oxazolone, according to the following score scale:
0 = no damage,
1 = hyperemia, no ulcers,
2 = hyperemia, thickening of the intestinal wall, no ulcers,
3 = one ulcer without thickening of the intestinal wall,
4 = 2 or more sites of ulceration or inflammation,
5 = 2 or more serious sites of ulceration and inflammation, or one site of ulceration/inflammation, extending >1 cm along the length of the colon, and
6-10 = damage covers >2 cm long the length of the colon, the score is increased by 1 for each additional 1 cm of involvement.

All data were analyzed by descriptive statistics: arithmetic mean (M) and standard error of the arithmetic mean (m). The Shapiro-Wilk test was used to check the normality of the distribution of the obtained experimental data. A normal distribution was analyzed with 1-way ANOVA (with Dunnett's post-analysis) to assess intergroup differences. A non-normal distribution was analyzed with 1-way ANOVA (with Tukey's post-analysis) to compare several groups. Differences were determined at a confidence level of 5%. The results of study are shown in Tables 1 and 2.

**Table 1. Effect of Compound 1 on damage of the colon wall in the murine model of acute oxazolone-induced ulcerative colitis (M ± m, n = 10)**

| Groups | Dose, mg/kg | n | The degree of large intestine wall damage, score |
|---|---|---|---|
| Intact | - | 10 | 0.00±0.00 |
| Control | - | 10 | 2.11±0.26* |
| Compound I | 0.3 | 10 | 1.70±0.30 |
| | 3 | 10 | 1.89±0.31 |
| | 30 | 10 | 1.40±0.16& |
| Prednisone | 10 | 10 | 1.80±0.25 |

| | | | |
|---|---|---|---|
| Note: * - statistical significance (P<0.05) vs. the intact group & - statistical significance (P<0.05) vs. the control group | | | |

**Table 2. Effect of Compound I on body weight of the animals in the mouse model of acute oxazolone-induced ulcerative colitis (M ± m, n = 10)**

| Group | Dose, mg/kg | n | Body weight, g | | | |
|---|---|---|---|---|---|---|
| | | | Before | 24 hours after | 48 hours after | 72 hours after |
| | | | administration of oxazolone | | | |
| Intact | - | 10 | 19.1±0.4 | 19.1±0.3 | 19.4±0.4 | 19.5±0.4 |
| Control | - | 10 | 18.9±0.5 | 17.2±0.3* | 16.6±0.3 | 16.1±0.3* |
| Compound I | 0.3 | 10 | 19.0±0.6 | 17.9±0.2 | 17.5±0.3 | 16.9±0.2 |
| | 3 | 10 | 18.7±0.4 | 17.9±0.3 | 17.6±0.2 | 16.7±0.2 |
| | 30 | 10 | 19.8±0.6 | 18.8±0.2& | 18.4±0.4& | 18.4±0.5& |
| Prednisone | 10 | 10 | 19.0±0.4 | 18.5±0.4 | 17.9±0.4 | 17.3±0.6 |

| | | | | | | |
|---|---|---|---|---|---|---|
| Note: * - statistical significance (P<0.05) vs. the intact group & - statistical significance (P<0.05) vs. the control group | | | | | | |

The results of the study showed that Compound I, when administered intragastrically, reduced the degree of damage of the colon wall and prevented weight loss in animals. Thus, Compound I had a therapeutic effect in the murine model of ulcerative colitis. Compound 1 was not inferior to prednisolone by intensity of action.

### Example 3. Study of the activity of Compound 1 in a guinea pig model of cough induced by inhalation of citric acid and IFN-γ.

The activity of Compound I in a model of cough in guinea pigs, which was induced by inhalation of citric acid and IFN-γ, was studied in accordance with the method [Am J Respir Crit Care Med. 2018. V. 198(7). P. 868-879].

In the study, guinea pigs of the Aguti line were used. All experimental animals were inhaled with a citric acid solution (0.3 M) prepared in physiological saline, for 8 minutes. The pathology control group and the groups receiving therapy were inhaled with IFN-γ (10 µg/kg) for 3 minutes at 7 hours before the inhalation of citrate. Compound 1 was administered intragastrically once, immediately after inhalation of IFN-γ, i.e. 7 hours before inhalation of the citric acid solution. The antitussive activity was evaluated by counting the number of coughing fits within 8 minutes from the start of inhalation of citric acid. All data were analyzed by descriptive statistics: arithmetic mean (M) and standard error of the arithmetic mean (m). The Shapiro-Wilk test was used to check the normality of the distribution of the obtained experimental data. A normal distribution was analyzed with 1-way ANOVA (with Dunnett's post-analysis) to assess intergroup differences. A non-normal distribution was analyzed with 1-way ANOVA (with Tukey's post-analysis) to compare several groups. Differences were determined at a confidence level of 5%. The results of study are shown in Tables 1 and 2.

The results of the study are given in Table 3.

The results of the study showed that Compound 1, when administered intragastrically, reduced the number of coughing movements. Thus, Compound I had a therapeutic effect in a guinea pig model of acute and subacute viral cough induced by inhalation of citric acid and IFN-γ. Table 3. Effect of Compound I on the number of cough movements in the guinea pig model of viral cough induced by inhalation of citric acid and IFN-γ (M ± m, n = 5).

| Groups | Dose, mg/kg | n | The number of coughing movements within 8 minutes |
|---|---|---|---|
| Intact | - | 5 | 0 |
| Citrate+placebo | - | 5 | 24.0±3.2 |
| Control (Citrate+IFN-γ) | - | 5 | 34.6±2.9 |
| Compound I | 1.4 | 5 | 19.2±2.7& |
| | 14 | 5 | 18.4±2.9& |

| | | | |
|---|---|---|---|
| Note: & - statistical significance (P <0.05) vs. the control group | | | |

## Claims

1. Use of a compound 1-(2-(1H-imidazol-4-yl)ethyl)piperidine-2,6-dione of the formula: 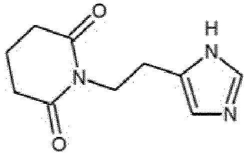 or a pharmaceutically acceptable salt thereof for suppressing aberrant interferon gamma signaling.

2. The use according to claim 1 for the treatment of a disease associated with aberrant interferon gamma signaling.

3. The use according to claim 2, wherein the disease associated with aberrant interferon gamma signaling is Sjögren's syndrome, dermatomyositis, systemic lupus erythematosus, or systemic sclerosis.

4. The use according to claim 1 for reducing or eliminating the onset of resistance to steroid therapy.

5. Use of a compound 1-(2-(1H-imidazol-4-yl)ethyl)piperidine-2,6-dione of the formula: 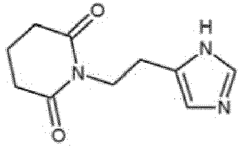 or a pharmaceutically acceptable salt thereof for the treatment of a disorder in steroid-resistant patient.

6. The use according to claim 5, wherein the disorder is asthma, rheumatoid arthritis, systemic lupus erythematosus, gastrointestinal diseases, and cough.

7. A pharmaceutical composition for suppressing aberrant interferon gamma signaling, wherein the pharmaceutical composition comprises a therapeutically effective amount of a compound 1-(2-(1H-imidazol-4-yl)ethyl)piperidine-2,6-dione of the formula: 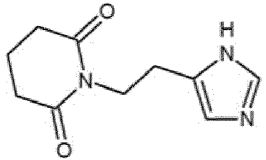 or a pharmaceutically acceptable salt thereof and at least one pharmaceutically acceptable carrier.

8. The pharmaceutical composition according to claim 7 for the treatment of a disease associated with aberrant interferon gamma signaling.

9. The pharmaceutical composition according to claim 8, wherein the disease associated with aberrant interferon gamma signaling is asthma, rheumatoid arthritis, systemic lupus erythematosus, gastrointestinal diseases, and cough.

10. The pharmaceutical composition according to claim 7 for preventing resistance to steroid therapy.

11. A pharmaceutical composition for treating a disorder in a steroid resistant patient, comprising a therapeutically effective amount of a compound 1-(2-(1H-imidazol-4-yl)ethyl) piperidine-2,6-dione of the formula: 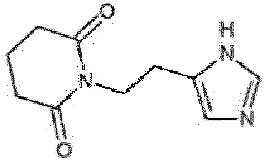 or a pharmaceutically acceptable salt thereof and at least one pharmaceutically acceptable carrier.

12. The pharmaceutical composition according to claim 11, wherein the disorder associated with aberrant interferon gamma signaling and with the development of steroid resistance is asthma, rheumatoid arthritis, systemic lupus erythematosus, gastrointestinal diseases, and cough.

13. Use of the compound as defined in claim 1 for the preparation of a pharmaceutical composition according to claim 7.

14. Use of the compound as defined in claim 5 for the preparation of a pharmaceutical composition according to claim 11.

15. A method of treating a disease associated with aberrant interferon gamma signaling, comprising administering a therapeutically effective amount of a compound 1-(2-(1H-imidazol-4-yl)ethyl)piperidine-2,6-dione of the formula: 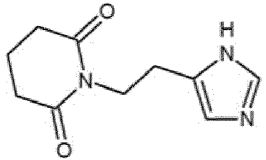 or a pharmaceutically acceptable salt thereof or a pharmaceutical composition according to claim 7 to a body.

16. The method according to claim 15, wherein the compound is administered at a dose of 10-200 mg/day.

17. The method according to any one of claims 15-16, wherein the compound is administered 1-2 times a day.

18. A method for treating a disorder in steroid resistant patients, comprising administering a therapeutically effective amount of a compound 1-(2-(1H-imidazol-4-yl)ethyl)piperidine-2,6-dione of the formula: 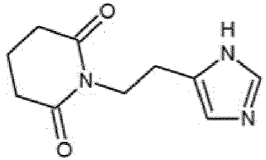 or a pharmaceutically acceptable salt thereof or a pharmaceutical composition according to claim 11 to the body.

19. The method according to claim 18, wherein the compound is administered at a dose of 10-200 mg/day.

20. The method according to any one of claims 18-19, wherein the compound is administered 1-2 times a day.

21. A combination for the treatment of a disorder in a steroid resistant patient, comprising a therapeutically effective amount of a compound 1-(2-(1H-imidazol-4-yl)ethyl)piperidine-2,6-dione of the formula: 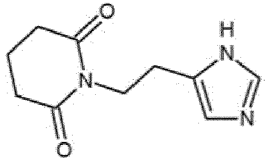 or a pharmaceutically acceptable salt thereof and a therapeutically effective amount of a steroid.

22. The combination according to claim 21, wherein the steroid is a corticosteroid.

23. The combination according to claim 21, wherein the disorder is asthma, rheumatoid arthritis, systemic lupus erythematosus, gastrointestinal diseases, and cough.

24. A method for treating a disorder in a steroid resistant patient, comprising administering a combination according to claim 21 to the body.

25. The method according to claim 24, wherein the compound and steroid are administered simultaneously or separately.

26. The method according to any one of claims 24-25, wherein the compound is administered at a dose of 10-200 mg/day.

27. The method according to any one of claims 24-26, wherein the compound is administered 1-2 times a day.
